# EUROPEAN PATENT APPLICATION

(11) **EP 4 797 226 A1**
(43) Date of publication of application: **26.08.2026**
(21) Application number: 23956055.0
(22) Date of filing: 18.10.2023
(51) Int. Cl.: G06V 40/14, A61B 5/1171, G06T 7/00

(54) **INFORMATION PROCESSING PROGRAM, INFORMATION PROCESSING METHOD, AND INFORMATION PROCESSING DEVICE**

(71) Applicant: FUJITSU LIMITED, Kawasaki-shi, Kanagawa 211-8588 (JP)
(72) Inventor: AOKI, Takahiro, Kawasaki-shi, Kanagawa 211-8588 (JP)
(74) Representative: Hoffmann Eitle
(86) International application number: PCT/JP2023/037672
(87) International publication number: WO 2025/083805

(57) **Abstract**

Authentication data that enables highly accurate biometric authentication is generated.

In an information processing program (1), a processing unit (3) generates a corrected image (12) by correcting, based on a biometric image (11) in which a predetermined body part is imaged, the posture of the body part in the biometric image (11). The processing unit (3) generates a feature image (13) by extracting a feature pattern indicating a feature of the shape of the body part from the corrected image (12). The processing unit (3) generates a corrected image (14) by correcting, based on the feature image (13), the posture of the feature pattern in the feature image (13). The processing unit (3) calculates authentication data (15) for biometric authentication based on the corrected image (14).

## Description

### Technical Field

The embodiments discussed herein relate to an information processing program, an information processing method, and an information processing apparatus.

### Background Art

In recent years, biometric authentication using various body parts has been used. For example, fingerprint authentication is often performed by bringing a body part (finger) into contact with a biometric sensor. On the other hand, in palm vein authentication and iris authentication, non-contact authentication is performed.

In the non-contact biometric authentication, the posture of the body part with respect to the biometric sensor may vary. The posture variation of the body part is a major factor causing an authentication error. Therefore, correcting the posture of the body part in the biometric image captured by the biometric sensor and calculating authentication data for authentication based on the corrected image are considered. There has also been proposed a technique for correcting the shape of a body part in addition to the posture of the body part in a biometric image.

Further, as a related technique, there has been proposed an authentication apparatus that illuminates an imaging portion of a biological body with light from a light source, detects transmitted light from the imaging portion, and captures an image of the biological body for authentication. This authentication apparatus optimizes the amount of light from the light source according to image information of the captured image.

### Citation List

### Patent Literature

PTL1: Japanese Laid-open Patent Publication No. 2016-170692
PTL2: Japanese Laid-open Patent Publication No. 2004-164651

### Summary of Invention

### Technical Problem

As described above, when the posture of the body part in the biometric image is corrected and the authentication data is calculated based on the corrected image, the correction accuracy of the posture may affect the accuracy of the biometric authentication (in particular, the accuracy rate of the personal authentication). In the posture correction process, there is a case where a correction error occurs and correction of the body part to a correct posture is not successively performed, and in this case, the accuracy of the biometric authentication deteriorates.

In one aspect, an object of the present disclosure is to provide: a storage medium storing an information processing program; an information processing method; and an information processing apparatus, which generate authentication data that enables highly accurate biometric authentication.

### Solution to Problem

In an aspect, there is provided an information processing program that causes a computer to execute a process including: generating a first corrected image by correcting, based on a biometric image in which a predetermined body part is imaged, a posture of the body part in the biometric image; generating a feature image by extracting a feature pattern indicating a feature of a shape of the body part from the first corrected image; generating a second corrected image by correcting, based on the feature image, a posture of the feature pattern in the feature image; and calculating authentication data for biometric authentication based on the second corrected image.

In an aspect, there is provided an information processing method executed by a computer that executes the same process as that based on the above-described information processing program.

Further, in an aspect, there is provided an information processing apparatus that executes the same process as that based on the above-described information processing program.

In addition, in an aspect, there is provided an information processing program that causes a computer to execute a process including: detecting, based on a biometric image in which a predetermined body part is imaged, a first deviation amount of a posture of the body part in the biometric image from a first reference state; generating a first corrected image by correcting the biometric image by the first deviation amount; generating a first feature image by extracting a feature pattern indicating a feature of a shape of the body part from the first corrected image; detecting a second deviation amount of a posture of the feature pattern in the first feature image from a second reference state; generating a second corrected image by correcting the biometric image by a sum of the first deviation amount and the second deviation amount; generating a second feature image by extracting the feature pattern from the second corrected image; and calculating authentication data for biometric authentication based on the second feature image.

In addition, in an aspect, there is provided an information processing method executed by a computer that executes the same process as that based on the above-described information processing program.

In addition, in an aspect, there is provided an information processing apparatus that executes the same process as that based on the above-described information processing program.

### Advantageous Effects of Invention

In one aspect, it is possible to generate authentication data that enables highly accurate biometric authentication.

The above and other objects, features and advantages of the present invention will become apparent from the following description taken in conjunction with the accompanying drawings which illustrate, by way of example, preferred embodiments of the invention.

### Brief Description of Drawings

[FIG. 1] FIG. 1 is a diagram illustrating a configuration example and a processing example of an information processing apparatus according to a first embodiment.
[FIG. 2] FIG. 2 is a diagram illustrating a configuration example of a biometric authentication system according to a second embodiment.
[FIG. 3] FIG. 3 is a diagram illustrating an example of a hardware configuration of an authentication terminal.
[FIG. 4] FIG. 4 is a diagram illustrating a comparative example of a process flow up to authentication data calculation.
[FIG. 5] FIG. 5 is a diagram illustrating a flow of a normalization process according to the second embodiment.
[FIG. 6] FIG. 6 is a diagram illustrating a configuration example of processing functions included in an authentication server.
[FIG. 7] FIG. 7 is a diagram illustrating a configuration example of processing functions included in the authentication terminal.
[FIG. 8] FIG. 8 is a diagram for describing a CNN and an STN.
[FIG. 9] FIG. 9 is a flowchart illustrating an example of a registration process in the authentication terminal.
[FIG. 10] FIG. 10 is a flowchart (part 1) illustrating an example of an authentication data generation process.
[FIG. 11] FIG. 11 is a flowchart (part 2) illustrating the example of the authentication data generation process.
[FIG. 12] FIG. 12 is a flowchart illustrating an example of a matching process in the authentication terminal.
[FIG. 13] FIG. 13 is a flowchart illustrating an example of a process of the authentication server.
[FIG. 14] FIG. 14 is a diagram illustrating a configuration example of processing functions of an authentication terminal according to a third embodiment.
[FIG. 15] FIG. 15 is a flowchart illustrating an example of an authentication data generation process according to the third embodiment.
[FIG. 16] FIG. 16 is a diagram illustrating a configuration example of processing functions of an authentication terminal according to a fourth embodiment.
[FIG. 17] FIG. 17 is a flowchart illustrating an example of an authentication data generation process according to the fourth embodiment.
[FIG. 18] FIG. 18 is a diagram illustrating a configuration example of processing functions of an authentication apparatus according to a fifth embodiment.

### Description of Embodiments

Hereinafter, embodiments of the present disclosure will be described with reference to the drawings.

### [First Embodiment]

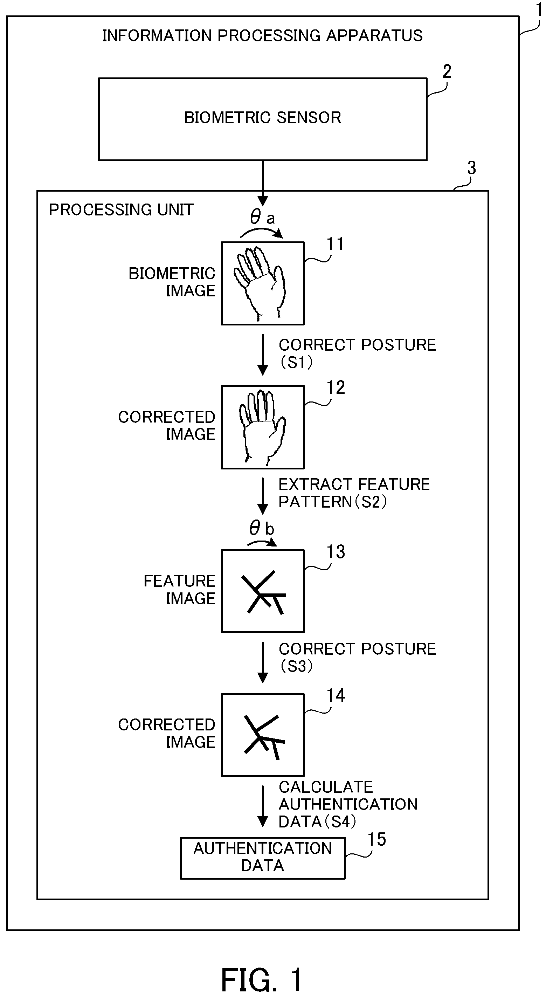

FIG. 1 is a diagram illustrating a configuration example and a processing example of an information processing apparatus according to a first embodiment. An information processing apparatus 1 illustrated in FIG. 1 is an apparatus for generating authentication data for biometric authentication. The information processing apparatus 1 includes a biometric sensor 2 and a processing unit 3.

The biometric sensor 2 captures an image of a predetermined body part of a user and outputs a biometric image. The biometric sensor 2 captures the biometric image without contact with a body part. As biometric authentication using such a non-contact sensor, for example, palm vein authentication, iris authentication, or the like is applicable. The biometric sensor 2 may be mounted inside the information processing apparatus 1 as illustrated in FIG. 1, or may be provided outside and connected to the information processing apparatus 1.

The processing unit 3 is, for example, a processor included in the information processing apparatus 1. In this case, the following processing by the processing unit 3 is implemented by, for example, the processor executing a predetermined program.

Based on a biometric image 11 captured by the biometric sensor 2, the processing unit 3 corrects the posture of the body part in the biometric image 11 and generates a corrected image 12 (step S1). In the example in FIG. 1, it is assumed that the processing unit 3 detects that the angle of the body part is deviated by an angle θa in the counterclockwise direction from a predetermined reference angle. In this case, the processing unit 3 generates the corrected image 12 by rotating the body part clockwise by the angle θa.

Next, the processing unit 3 generates a feature image 13 by extracting a feature pattern indicating a feature of the shape of the body part from the generated corrected image 12 (step S2). For example, when vein authentication is applied, a vein pattern indicating the blood vessel shapes of veins is extracted as a feature pattern.

Next, based on the generated feature image 13, the processing unit 3 corrects the posture of the feature pattern in the feature image 13 and generates a corrected image 14 (step S3). In the example in FIG. 1, it is assumed that the processing unit 3 detects that the angle of the feature pattern deviates from a predetermined reference angle by an angle θb in the counterclockwise direction. In this case, the processing unit 3 generates the corrected image 14 by rotating the feature pattern clockwise by the angle θb.

Next, the processing unit 3 calculates authentication data 15 for biometric authentication based on the generated corrected image 14 (step S4). The generated authentication data 15 is registered in a database as registration data corresponding to the target user, for example. Alternatively, the authentication data 15 is matched against registration data registered in a database.

According to the above-described processes, image correction is executed at two stages in step S1 and step S3. Thus, even when a correction error of the posture occurs in the image correction in step S1, it is possible to correct the posture more accurately by the image correction in step S3. Therefore, the correction accuracy of the posture is improved as a whole. As a result, the authentication data 15 that enables highly accurate biometric authentication is generated.

In particular, in the image correction in step S1, for example, the shape of a body part such as a palm is detected from the biometric image 11, and the deviation amount of the posture is detected from the detection result of the shape. In this case, since the amount of information for detecting the deviation amount of the posture (for example, information comparable with a reference amount serving as a reference for determining the deviation) is less, the detection accuracy of the deviation amount is low, and as a result, the correction accuracy of the posture is also low.

On the other hand, in the image correction in step S3, most of the feature pattern of the body part included in the feature image 13 is often used to detect the deviation amount of the posture, and the amount of information used for the detection is greater than that in the image correction in step S1. Therefore, the detection accuracy of the deviation amount is high, and as a result, the correction accuracy of the posture is also high. Therefore, the correction error of the posture generated by the image correction in step S1 is canceled by the image correction in step S3, and the correction accuracy as a whole is improved.

Further, in step S1, a maximum posture correction amount, or a maximum posture correction value, may be set. In this case, for example, the processing unit 3 may detect the deviation amount of the body part from the reference state from the captured biometric image 11. If the detected deviation amount is equal to or less than the maximum value, the processing unit 3 may generate the corrected image 12 by correcting the biometric image 11. If the detected deviation amount is greater than the maximum value, the processing unit 3 may output a message requesting the user to correct the posture of the body part with respect to the biometric sensor 2. In this case, the processing unit 3 acquires a biometric image newly captured by the biometric sensor 2 after the message is output. If the deviation amount of the body part from the reference state is equal to or less than the maximum value in the acquired biometric image, the processing unit 3 generates the corrected image 12 by correcting this biometric image.

In the above-described case, as the maximum value in step S1 is set greater, the deviation amount of the body part becomes equal to or less than the maximum value within a shorter time on average. On the other hand, as the maximum value is set greater and the image correction amount in step S1 becomes greater, the correction error may become greater. However, according to the above-described process, after the image correction in step S1 is executed, the image correction in step S3 is additionally executed. Thus, it is possible to increase the allowable amount of the correction error that may occur in step S1. Therefore, it is possible to set the maximum value to be larger and shorten the time needed until the deviation amount of the body part becomes equal to or less than the maximum value. As a result, it is possible to shorten the entire processing time from when the body part is held over the biometric sensor 2 to when the authentication data 15 is calculated, without deteriorating the correction accuracy of the image.

### [Second Embodiment]

Next, a system in which vein authentication using veins of a palm is executed as an example of biometric authentication will be described.

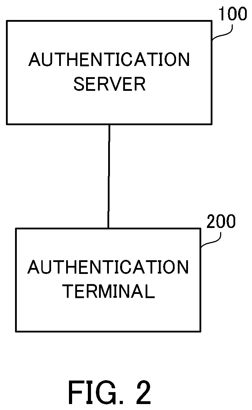

FIG. 2 is a diagram illustrating a configuration example of a biometric authentication system according to a second embodiment. As illustrated in FIG. 2, the biometric authentication system includes an authentication server 100 and an authentication terminal 200. The authentication server 100 and the authentication terminal 200 are connected to each other via a network.

The authentication terminal 200 is an example of the information processing apparatus 1 illustrated in FIG. 1, and is a client apparatus corresponding to the authentication server 100. The authentication terminal 200 includes a biometric sensor for acquiring biometric information of a user. In the present embodiment, information on veins of a palm is used as the biometric information. The authentication terminal 200 generates authentication data used for authentication by performing image processing on a biometric image captured by the biometric sensor. The authentication data is feature data of a body part for identifying the user, and is calculated as, for example, a feature vector.

The authentication terminal 200 generates authentication data for registration (registration data) from biometric information of a user to be registered, transmits the generated registration data to the authentication server 100, and requests registration in a database. In addition, the authentication terminal 200 generates authentication data for matching (matching data) from biometric information of the user to be authenticated, transmits the generated matching data to the authentication server 100, and requests matching against the registration data registered in the database. The authentication terminal 200 receives an authentication result from the authentication server 100 as a response to the matching request, and executes processing according to the authentication result.

The authentication server 100 registers the registration data transmitted from the authentication terminal 200 in a database. Further, the authentication server 100 matches the matching data transmitted from the authentication terminal 200 against the registration data registered in the database, and transmits an authentication result to the authentication terminal 200.

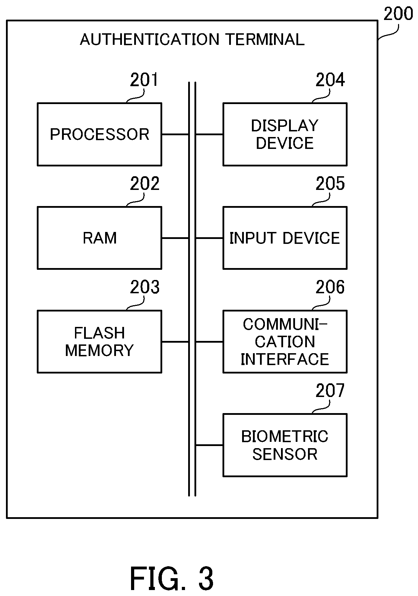

FIG. 3 is a diagram illustrating an example of a hardware configuration of the authentication terminal. The authentication terminal 200 is implemented as, for example, a computer as illustrated in FIG. 3. As illustrated in FIG. 3, the authentication terminal 200 includes a processor 201, a random access memory (RAM) 202, a flash memory 203, a display device 204, an input device 205, a communication interface 206, and a biometric sensor 207.

The processor 201 comprehensively controls the entire authentication terminal 200. The processor 201 is, for example, a central processing unit (CPU), a micro processing unit (MPU), a digital signal processor (DSP), an application specific integrated circuit (ASIC), or a programmable logic device (PLD). The processor 201 may be a combination of two or more elements of a CPU, an MPU, a DSP, an ASIC, and a PLD. The processor 201 is an example of the processing unit 3 illustrated in FIG. 1.

The RAM 202 is used as a main storage device of the authentication terminal 200. The RAM 202 temporarily stores at least part of the operating system (OS) programs and firmware programs to be executed by the processor 201. The RAM 202 stores various data that the processor 201 needs for processing.

The flash memory 203 is used as an auxiliary storage device of the authentication terminal 200. The flash memory 203 stores OS programs, firmware programs, and various data. As the auxiliary storage device, another type of nonvolatile storage device such as a hard disk drive (HDD) may be used.

The display device 204 displays an image in accordance with an instruction from the processor 201. Examples of the display device 204 include a liquid crystal display and an organic EL display.

The input device 205 receives an input operation, and transmits a signal corresponding to the input operation to the processor 201. The input device 205 includes operation buttons, a touch panel, and the like.

The communication interface 206 transmits and receives data to and from other devices such as the authentication server 100 via a network.

The biometric sensor 207 includes a light emitting unit and an imaging unit (which are not illustrated). The light emitting unit illuminates a body part (palm) with near-infrared light. The imaging unit captures a biometric image by receiving reflected light of the near-infrared light from the body part, and transmits data of the biometric image to the processor 201. The biometric sensor 207 is an example of the biometric sensor 2 illustrated in FIG. 1.

With the hardware configuration described above, it is possible to implement the processing functions of the authentication terminal 200. It is also possible to implement the authentication server 100 as a computer including a processor and a storage device.

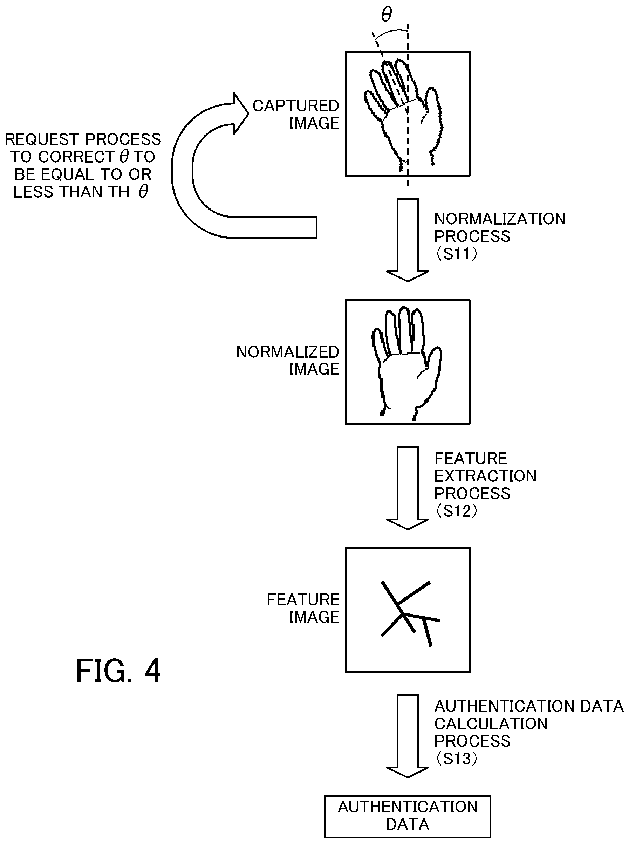

FIG. 4 is a diagram illustrating a comparative example of a process flow up to authentication data calculation.

It is desirable that a body part of the authentication target be captured in a constant posture in a biometric image captured by a biometric sensor. However, in practice, the user does not always keep the body part at a correct angle with respect to the biometric sensor. Therefore, the captured biometric image often includes a posture variation of the body part. In particular, in non-contact biometric authentication such as palm vein authentication or iris authentication, a posture variation of a body part is likely to occur. Such a posture variation is a main cause of a biometric authentication error, and reducing the posture variation is an important factor for improving the accuracy of the authentication (in particular, improving the accuracy rate of the personal authentication).

Therefore, a "normalization process" for correcting the posture of the body part (the palm in the present embodiment) in the image to a correct posture is executed on the captured image obtained from the biometric sensor 207 (step S11). FIG. 4 illustrates a case in which the rotation angle of the palm is corrected. In the normalization process, for example, the contour of the palm is detected from the captured image, and an angle θ indicating the deviation amount of the rotation angle from a reference angle corresponding to a correct posture is calculated based on an edge angle of the contour. Next, the captured image is rotated by the angle θ such that the deviation of the angle is canceled by affine transformation or the like, and a normalized image (corrected image), which is a captured image obtained after the normalization, is generated.

Next, a feature extraction process for extracting a vein pattern from the generated normalized image is executed (step S12). In the feature extraction process, for example, a vein enhancement process based on a band-pass filter or the like is applied to the normalized image, and a noise removal process is further applied. A feature image is generated by such feature extraction process, and authentication data for registration or matching is calculated using a predetermined calculation method based on the feature image (step S13).

By applying the feature extraction process, it is possible to obtain an image having a large amount of information such as a vein pattern. On the other hand, since a plurality of filters are often applied to the entire image in the feature extraction process, the processing time is generally long. Therefore, it is desirable to minimize the number of times the feature extraction process is called.

In the above processes, since the authentication data is calculated based on the captured image in which the posture of the palm has been corrected by the normalization process in step S11, it is possible to calculate authentication data that does not cause an authentication error easily. It is possible to say that the higher the correction accuracy of the posture by the normalization process is, the more the accuracy of the biometric authentication is improved. This means that when the performance of the normalization process is low, the accuracy of the biometric authentication is also low.

Further, in the normalization process in step S11, an upper limit threshold TH_θ is set for the correctable posture correction amount. Then, in a case where the absolute value of the angle θ calculated from the captured image exceeds the threshold TH_θ, a request process for requesting the user to correct the posture of the palm is executed. In the request process, the user is notified of a message such as "please straighten your palm" by an image or voice.

The angle θ is calculated again from a captured image captured by the biometric sensor 207 after the execution of the request process. If the absolute value of the angle θ is equal to or less than the threshold TH_θ, the feature extraction process (step S12) for extracting a vein pattern from the captured image is executed.

In the image correction in the normalization process in step S11, a correction error may occur, and in some cases, the posture of the palm in the normalized image is not corrected to a correct posture. In these cases, the accuracy of the authentication process using the authentication data based on the normalized image deteriorates. Therefore, in order to improve the accuracy of the authentication process, the image correction accuracy in the normalization process needs to be improved.

In particular, although the image correction in step S11 is performed on the captured image, the correction of the posture using the captured image has the following problems. In the image correction on the captured image, for example, the shape of a body part such as a palm is detected from the captured image, and the deviation amount of the posture is detected from the detection result of the shape. In this case, since the amount of information for detecting the deviation amount of the posture (for example, information comparable with a reference amount serving as a reference for determining the deviation) is less, there is a problem in that the detection accuracy of the deviation amount is low, and as a result, the correction accuracy of the posture is also low.

In addition, in the normalization process, in general, as the deviation amount of the posture increases (that is, as the correction amount increases), a calculation error of the deviation amount (that is, the angle θ) or an error of the image correction may increase. In the normalization process in step S11, since it is possible to suppress the correction amount by requesting the user so that the absolute value of the detected angle θ becomes equal to or less than the threshold TH_θ, and thus, it is possible to improve the accuracy of the correction process. However, although it is possible improve the correction accuracy by decreasing the threshold TH_θ, the requesting time needed until the absolute value of the angle θ becomes equal to or less than the threshold TH_θ becomes longer on average. As a result, the entire time from when the user holds the palm over the biometric sensor 207 to when the process of registration or matching of authentication data is completed becomes longer. On the other hand, although it is possible to shorten the entire time on average by increasing the threshold TH_θ, since the absolute value of the angle θ increases, the correction accuracy deteriorates.

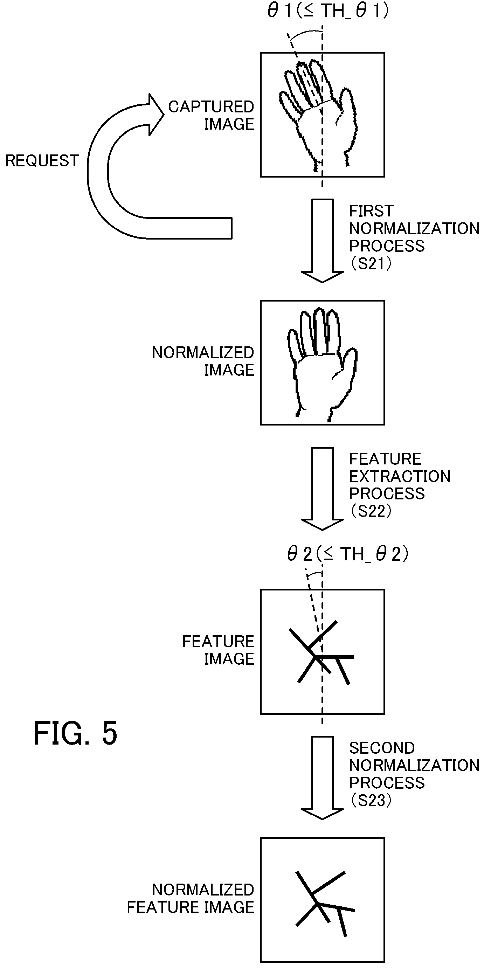

FIG. 5 is a diagram illustrating a flow of the normalization process according to the second embodiment. In the present embodiment, the authentication terminal 200 executes the normalization process in two stages to improve the accuracy of the posture correction by the normalization process.

The authentication terminal 200 first executes a first normalization process on a captured image obtained from the biometric sensor 207 (step S21). FIG. 5 illustrates an example in which the rotation angle of the palm is corrected as in FIG. 4. In this case, the authentication terminal 200 calculates an angle θ1 indicating the deviation amount of the rotation angle from the reference angle based on the contour of the palm in the captured image. Further, an upper limit threshold TH_θ1 is set for the correctable angle correction amount. If the absolute value of the calculated angle θ1 exceeds the threshold TH_θ1, the authentication terminal 200 executes a requesting process of requesting the user to correct the posture of the palm. When the requesting process is executed, the authentication terminal 200 acquires a captured image obtained from the biometric sensor 207 again, and calculates the angle θ1 from the captured image.

If the absolute value of the angle θ1 is equal to or less than the threshold TH_θ1, the authentication terminal 200 generates a normalized image (first corrected image) in which the posture of the palm has been corrected by the angle θ1 such that the deviation of the rotation angle is canceled. Next, the authentication terminal 200 executes a feature extraction process for extracting a vein pattern from the generated normalized image (step S22). As a result, a feature image is generated.

Next, the authentication terminal 200 executes a second normalization process on the generated feature image (step S23). In this process, the authentication terminal 200 calculates an angle θ2 indicating the deviation amount of the rotation angle from the reference angle for the vein pattern. Further, an upper limit threshold TH_θ2 is set for the correctable angle correction amount. If the absolute value of the calculated angle 82 is equal to or less than the threshold TH_θ2, the authentication terminal 200 generates a normalized feature image (second corrected image) in which the posture of the palm has been corrected by the angle θ2 such that the deviation of the rotation angle is canceled. Thereafter, the authentication terminal 200 calculates authentication data for registration or matching based on the normalized feature image.

According to the above-described processes, by executing the normalization process in two stages, even when a correction error of the posture occurs in the first normalization process, it is possible to correct the posture more accurately by the second normalization process. For this reason, it is possible to improve the correction accuracy of the posture as a whole, and as a result, it is possible to generate authentication data that enables highly accurate biometric authentication.

In particular, as described above, in the image correction on a captured image, since the amount of information usable to detect the deviation amount of the posture is less, there is a problem in that the detection accuracy of the deviation amount is low, and as a result, the correction accuracy of the posture is also low. On the other hand, in the second normalization process, image correction is performed on the feature image. In the image correction on the feature image, most of the feature pattern of the body part included in the feature image is often used to detect the deviation amount of the posture. For example, when the second normalization process is executed using a spatial transformer network (STN) to be described later, the entire feature pattern in the input feature image is used to detect the deviation amount of the posture. Thus, in the image correction on the feature image, since the amount of information used for detecting the deviation amount of the posture is greater than that in the image correction on the captured image, the detection accuracy of the deviation amount is high, and as a result, the correction accuracy of the posture is also high.

In the processes in FIG. 5, after the image correction on the captured image is executed by the first normalization process, the highly accurate image correction is performed on the feature image by the second normalization process. Therefore, it is possible to cancel the correction error of the posture generated in the first normalization process by the second normalization process, and it is possible to improve the correction accuracy as a whole.

Further, for example, since the feature image to be subjected to the second normalization process is generated based on the normalized image subjected to the first normalization process, the rotation angle θ2 in the second normalization process is usually less than the rotation angle θ1 in the first normalization process. Therefore, the second normalization process is more likely to perform highly accurate correction than the first normalization process, and as a result, it is possible to improve the correction accuracy as a whole.

Further, it is possible to make the processing procedure of the first normalization process simpler than that of the second normalization process. For example, in the first normalization process, an image with a reduced resolution may be generated from the captured image, and the angle θ1 may be calculated from the image. Such simplification of the first normalization process enables reduction of the time needed for the first normalization process including the requesting process. Thus, it is possible to improve the correction accuracy in the normalization process while suppressing an increase in the overall time needed until the authentication data is calculated.

Further, since the angle θ2 is more likely to be less than the angle θ1 as described above, it is possible to make the threshold TH_θ1 in the first normalization process greater than the threshold TH_θ2 in the second normalization process. By making the threshold TH_θ1 relatively large in this manner, it is possible to shorten the average time needed for the requesting process. On the other hand, by making the threshold TH_θ2 relatively less, it is possible to reduce the processing load of the posture detection in the second normalization process, and it is possible to shorten the processing time.

Further, by making the threshold TH_θ1 relatively large as described above, it is possible to make the threshold TH_θ1 greater than the threshold TH_θ used in the case where the one-stage normalization process is executed as illustrated in FIG. 4, and therefore, it is possible to shorten the average time needed for the requesting process. As a result, it is possible to shorten the entire time from when the user holds the palm over the biometric sensor 207 to when the registration or matching process of the authentication data is completed, without deteriorating the correction accuracy in the normalization process.

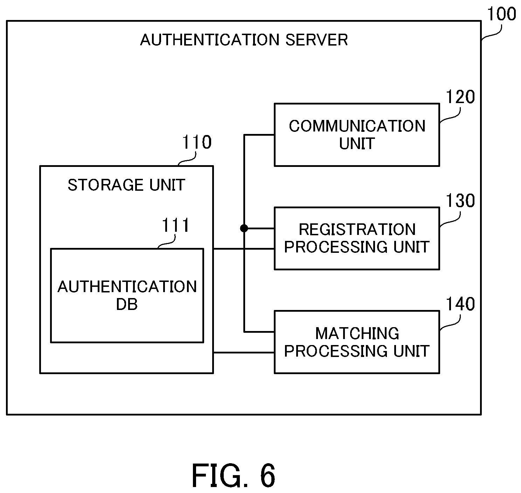

FIG. 6 is a diagram illustrating a configuration example of processing functions included in the authentication server. The authentication server 100 includes a storage unit 110, a communication unit 120, a registration processing unit 130, and a matching processing unit 140.

The storage unit 110 is a storage area allocated in a storage device (not illustrated) included in the authentication server 100. The storage unit 110 stores an authentication database (DB) 111. In the authentication database 111, registration data of authentication target users is registered in association with user IDs for identifying their respective users.

The processes of the communication unit 120, the registration processing unit 130, and the matching processing unit 140 are implemented by, for example, a processor (not illustrated) included in the authentication server 100 executing a predetermined application program.

The communication unit 120 communicates with the authentication terminal 200. For example, upon receiving a registration request together with authentication data and a user ID from the authentication terminal 200, the communication unit 120 passes the authentication data and the user ID to the registration processing unit 130. For example, in a case where one-to-one authentication is performed, when the communication unit 120 receives a matching request together with authentication data and a user ID from the authentication terminal 200, the communication unit 120 passes the authentication data and the user ID to the matching processing unit 140. Thereafter, the communication unit 120 transmits an authentication result obtained from the matching processing unit 140 to the authentication terminal 200.

The registration processing unit 130 registers authentication data (registration data) in the authentication database 111 in association with a user ID. The matching processing unit 140 reads the registration data corresponding to a user ID from the authentication database 111, matches the registration data against received authentication data (matching data), and passes an authentication result indicating whether or not the authentication is successful to the communication unit 120.

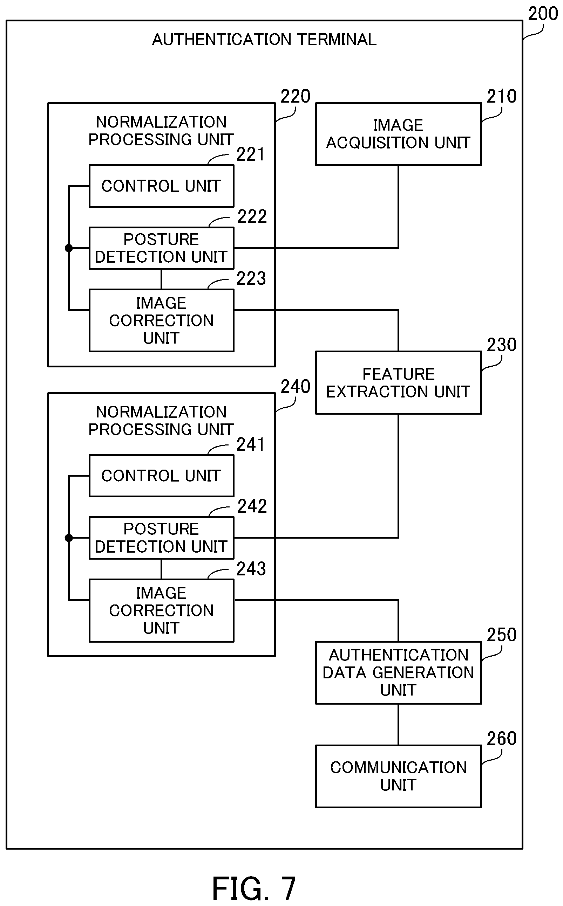

FIG. 7 is a diagram illustrating a configuration example of processing functions included in the authentication terminal. The authentication terminal 200 includes an image acquisition unit 210, a normalization processing unit 220, a feature extraction unit 230, a normalization processing unit 240, an authentication data generation unit 250, and a communication unit 260. The processes of the image acquisition unit 210, the normalization processing unit 220, the feature extraction unit 230, the normalization processing unit 240, the authentication data generation unit 250, and the communication unit 260 are implemented by, for example, the processor 201 executing a predetermined firmware program.

The image acquisition unit 210 acquires a biometric image captured by the biometric sensor 207.

The normalization processing unit 220 performs the first normalization process on the biometric image. In the normalization process, a requesting process for requesting the user to change the posture of the palm held over the biometric sensor 207 to an appropriate posture is executed.

The normalization processing unit 220 includes a control unit 221, a posture detection unit 222, and an image correction unit 223. The control unit 221 controls the entire first normalization process, and executes a process of comparing a posture detection result with a threshold and a process of outputting a request message. The posture detection unit 222 detects the deviation amount of the posture of a palm from a biometric image. The image correction unit 223 corrects the biometric image such that the posture of the palm is optimized according to the detected deviation amount, and generates a normalized image.

The feature extraction unit 230 performs a feature extraction process for extracting a vein pattern on the normalized image, to generate a feature image.

The normalization processing unit 240 executes the second normalization process on the feature image. The normalization processing unit 240 includes a control unit 241, a posture detection unit 242, and an image correction unit 243. The control unit 241 controls the entire second normalization process, and executes a comparison process between a posture detection result and a threshold. The posture detection unit 242 detects the deviation amount of the posture of a vein pattern obtained from the feature image. The image correction unit 243 corrects the feature image such that the posture of the vein pattern is optimized according to the detected deviation amount.

The authentication data generation unit 250 generates authentication data for registration or matching by executing a predetermined calculation on the feature image corrected by the second normalization process.

The communication unit 260 transmits the generated authentication data to the authentication server 100 together with the user ID corresponding to the biometric image, and requests data registration or data matching. Further, the communication unit 260 receives an authentication result from the authentication server 100 when the data matching is requested.

Here, it is possible to implement the process of the authentication data generation unit 250 by using a convolutional neural network (CNN). In this case, it is possible to implement the processes of the posture detection unit 242 and the image correction unit 243 of the normalization processing unit 240 by using an STN placed before the CNN.

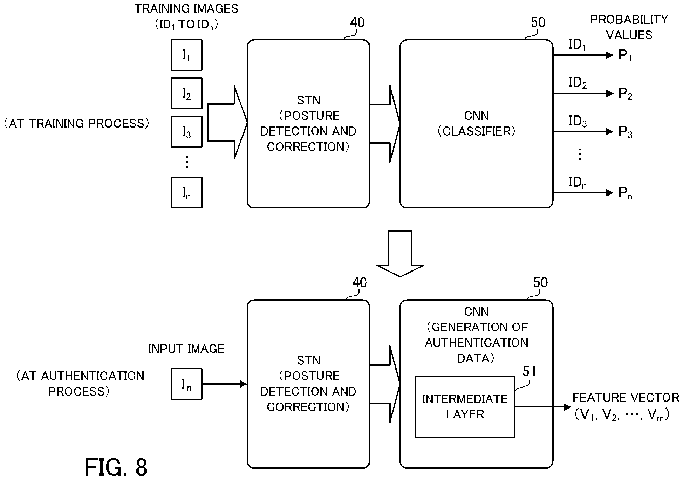

FIG. 8 is a diagram for describing the CNN and the STN. When an input image Iᵢₙ is input, a CNN 50 illustrated in FIG. 8 outputs an m-dimensional feature vector (V₁, V₂, ···, Vₘ) as authentication data. The CNN is generated by, for example, the following machine learning.

For training of the CNN 50, n training images I₁ to Iₙ are prepared. The training images I₁ to Iₙ are biometric images obtained by capturing images of palms of different people. The training images I₁ to Iₙ are associated with ID₁ to IDₙ, respectively, as identification information for identifying the imaging target people. By using such training images I₁ to Iₙ as training data and using ID₁ to IDₙ as correct answer data, a classifier with n categories that classifies an input image into one of the n people corresponding to the training images I₁ to Iₙ is trained. As a result, when an image is input, the CNN 50 that calculates the probability values P₁ to Pₙ corresponding to ID₁ to IDₙ is generated.

The authentication terminal 200 generates authentication data using the CNN 50 generated as described above. The CNN 50 includes an input layer, a plurality of intermediate layers (hidden layers), and an output layer. When the input image Iᵢₙ is input, the CNN 50 outputs m weight coefficients output from a predetermined intermediate layer 51 as an m-dimensional feature vector (V₁, V₂, ···, Vₘ). The output feature vector (V₁, V₂, ···, Vₘ) is information indicating the feature of the input image Iᵢₙ.

The feature vectors (V₁, V₂, ···, Vₘ) may be output from two or more predetermined intermediate layers. For example, n = 10000 and m = 512.

In addition, an STN having a posture correction function may be placed at a previous stage of the CNN for image classification. In the example in FIG. 8, an STN 40 is placed at a previous stage of the CNN 50. In the authentication process, when the input image Iᵢₙ is input to the STN 40, the STN 40 detects the deviation amount of the posture and corrects the input image Iᵢₙ by the deviation amount. The input image Iᵢₙ thus normalized is input to the CNN 50, and the feature vector (V₁, V₂, ···, Vₘ) is calculated. Such an STN 40 is generatable simultaneously with the CNN 50 by machine learning using the training images I₁ to Iₙ.

In the authentication terminal 200, the STN 40 is applicable as the posture detection unit 242 and the image correction unit 243 of the normalization processing unit 240, and the CNN 50 is applicable as the authentication data generation unit 250. In this case, at the time of training of the STN 40 and the CNN 50, feature images from which vein patterns are extracted are used as the training images I₁ to Iₙ.

As described above, the STN 40 is generated by machine learning using a large number of feature images as training data. Therefore, when the STN 40 is applied as the posture detection unit 242 and the image correction unit 243, the entire vein pattern included in the input feature image is used to detect the deviation amount of the posture. Therefore, the amount of information used for detecting the deviation amount is larger than that in the process of the normalization processing unit 220 in which correction is performed on the captured image. As a result, the detection accuracy of the deviation amount is increased, and the accuracy of the image correction is also increased.

Next, processes of the authentication server 100 and the authentication terminal 200 will be described with reference to flowcharts.

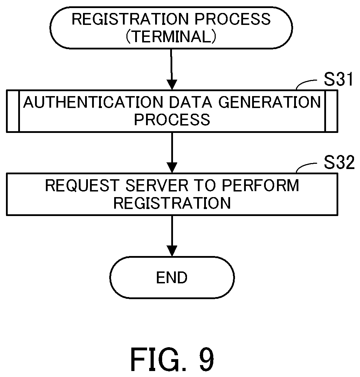

FIG. 9 is a flowchart illustrating an example of the registration process in the authentication terminal.

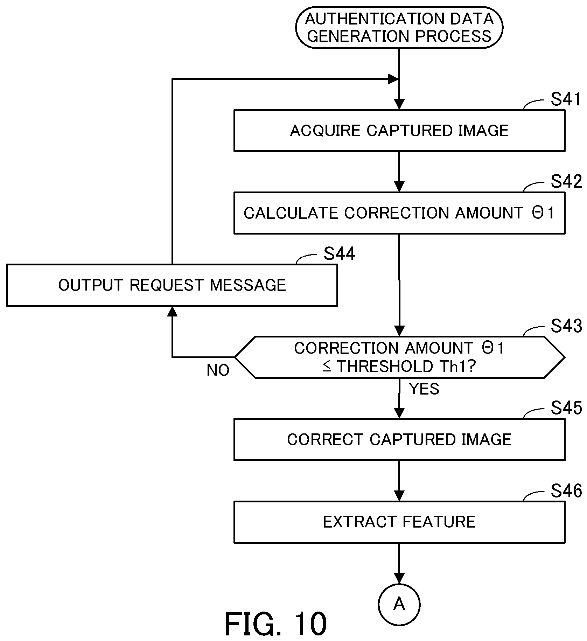

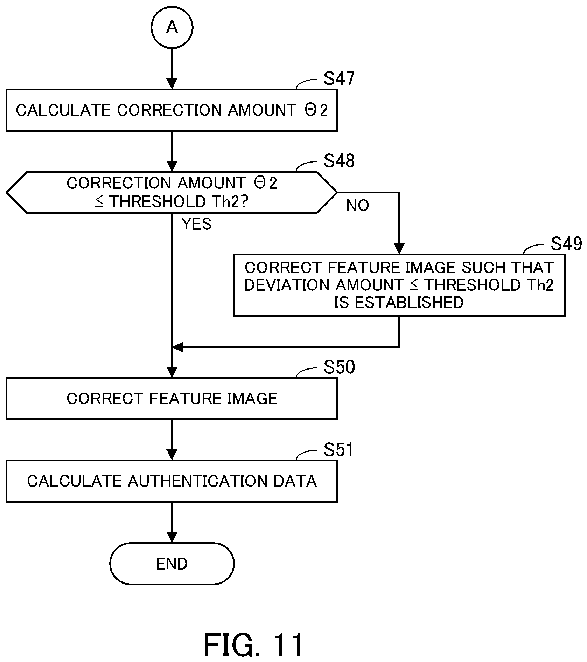

[Step S31] An authentication data generation process is executed based on a biometric image captured by the biometric sensor 207. This process will be described in detail below with reference to FIGS. 10 and 11.

[Step S32] The communication unit 260 transmits the generated authentication data to the authentication server 100 together with a corresponding user ID, and requests registration of the data in the authentication database 111.

FIGS. 10 and 11 are flowcharts illustrating an example of the authentication data generation process. The process in FIGS. 10 and 11 correspond to step S31 in FIG. 9. In FIGS. 10 and 11, it is assumed that not only the rotation angles of the palm and the vein pattern but also the position and the scale are corrected in the normalization process.

[Step S41] The image acquisition unit 210 acquires a captured image (biometric image) captured by the biometric sensor 207 together with a user ID that identifies the imaging target user.

[Step S42] The posture detection unit 222 of the normalization processing unit 220 calculates a correction amount Θ1 indicating the deviation amount of the posture of the palm in the captured image from a predetermined reference state (first reference state). The correction amount Θ1 includes an angle θ1 indicating the rotation angle deviation amount, translation amounts (dx1, dy1) indicating the positional deviation amounts, and an enlargement/reduction ratio s1 indicating the scale deviation amount.

Regarding the rotation angle, the posture detection unit 222 detects, for example, the contour of the palm in the captured image, and detects a vertical edge of the contour. The posture detection unit 222 calculates an average value of the angles of a longitudinal edge. The individual angle is detected as an angular difference from a predetermined reference angle (for example, the vertical direction) indicating a correct posture, and an average value of the angles is calculated as the angle θ1.

Regarding the position, the posture detection unit 222 calculates, for example, the centroid coordinates of the palm region in the captured image, and calculates differences in x and y coordinates between a predetermined reference position and the centroid coordinates in the captured image, as the translation amounts (dx1, dy1).

Regarding the scale, for example, the posture detection unit 222 calculates the area of the palm region in the captured image, and calculates the ratio between the calculated area and a predetermined reference area as the enlargement/reduction ratio s1.

[Step S43] The control unit 221 of the normalization processing unit 220 determines whether the calculated correction amount Θ1 is equal to or less than a predetermined threshold Th1. If the correction amount Θ1 exceeds the threshold Th1, the process proceeds to step S44. If the correction amount Θ1 is equal to or less than the threshold Th1, the process proceeds to step S45.

[Step S44] The control unit 221 outputs a request message requesting the user to correct the posture of the palm with respect to the biometric sensor 207 by an image or sound. Thereafter, the process proceeds to step S41, and a newly captured image is acquired.

[Step S45] The image correction unit 223 of the normalization processing unit 220 generates a normalized image by correcting the captured image by the correction amount Θ1 such that the deviation from the threshold Th1 is canceled.

As the threshold Th1 for the correction amount Θ1, in practice, a threshold Th_θ1 for the rotation angle, thresholds Th_dx1 and Th_dy1 for the position, and thresholds Th_s1_min and Th_s1_max for the scale are set. In step S43, threshold determination is performed for each of the rotation angle, the position, and the scale. If any one of these determination conditions based on their respective thresholds is not satisfied, the process proceeds to step S44.

For example, the determination condition for the rotation angle is that the absolute value of the calculated angle θ1 is equal to or less than the threshold Th_θ1. The determination condition for the position is that the absolute value of the translation amount dx1 in the x direction is equal to or less than the threshold Th_dx1 and the absolute value of the translation amount dy1 in the y direction is equal to or less than the threshold Th_dy1. The determination condition for the scale is that the calculated enlargement/reduction ratio s1 is included in the range from the threshold Th_s1_min to the threshold Th_s1_max. The threshold of the scale is set to Th_s1_min < Th_s1_max, for example, Th_s1_min = 0.9 and Th_s1_max = 1.1.

In step S44, a request message related to an item whose determination condition is not satisfied may be output. For example, if the determination condition for the rotation angle is not satisfied, a request message for requesting the user to correct the rotation angle of the palm may be output.

In step S45, image correction is performed such that the deviation is canceled from the reference value by the calculated angle θ1, translation amounts (dx1, dy1), and enlargement/reduction ratio s1.

[Step S46] The feature extraction unit 230 performs a feature extraction process for extracting a vein pattern on the generated normalized image, to generate a feature image. In the feature extraction process, for example, a vein enhancement process by a band-pass filter or the like is applied to the normalized image, and a noise removal process is further applied.

[Step S47] The posture detection unit 242 of the normalization processing unit 240 calculates a correction amount Θ2 indicating the deviation amount of the posture of the vein pattern in the feature image from a predetermined reference state (second reference state). As is the case with the correction amount Θ1, the correction amount Θ2 includes an angle θ2 indicating the rotation angle deviation amount, translation amounts (dx2, dy2) indicating the positional deviation amounts, and a scaling factor s2 indicating the scale deviation amount.

The angle θ2 and the translation amounts (dx2, dy2) are calculated as difference values from predetermined reference values defined for the respective items. The enlargement/reduction ratio s2 is calculated as a ratio to a predetermined reference area. As an example, these reference values and reference area are generated by training of the STN 40, and the angle θ2, the translation amounts (dx2, dy2), and the enlargement/reduction ratio s2 are output from the STN 40 that has received the input of a feature image.

[Step S48] The control unit 241 of the normalization processing unit 240 determines whether the calculated correction amount Θ2 is equal to or less than a predetermined threshold Th2. If the correction amount Θ2 exceeds the threshold Th2, the process proceeds to step S49. If the correction amount Θ2 is equal to or less than the threshold Th2, the process proceeds to step S50.

[Step S49] The process proceeds to step S49, for example, when the normalization process in the normalization processing unit 220 is not appropriately performed and when the deviation amount from the threshold Th2 exceeds the upper limit amount correctable by the normalization processing unit 240. In this case, the image correction unit 243 of the normalization processing unit 240 corrects the feature image such that the deviation amount from the threshold Th1 becomes equal to or less than the threshold Th2. This image correction may be executed by, for example, the image correction unit 223 of the normalization processing unit 220.

[Step S50] The image correction unit 243 of the normalization processing unit 240 generates a normalized image by correcting the feature image by the correction amount Θ2 such that the deviation from the threshold Th2 is canceled. When step S49 is executed, the image correction unit 243 corrects the feature image corrected in step S49 by the upper limit value (= threshold Th2) of the correction amount Θ2.

As is the case with the correction amount Θ1, as the threshold Th2 for the correction amount Θ2, in practice, the threshold Th_θ2 for the rotation angle, thresholds Th_dx2 and Th_dy2 for the position, and thresholds Th_s2_min and Th_s2_max for the scale are set. In step S48, threshold determination is performed for each of the rotation angle, the position, and the scale. For all of these items, if the determination conditions based on their respective thresholds are satisfied, in step S50, image correction is performed such that the deviation from the reference value is canceled by the calculated angle θ2, translation amounts (dx2, dy2), and enlargement/reduction ratio s2. On the other hand, if any one of these determination conditions for the rotation angle, the position, and the scale is not satisfied, the process proceeds to step S49.

[Step S51] The authentication data generation unit 250 calculates authentication data for registration or matching by performing a predetermined calculation using the feature image corrected in step S50.

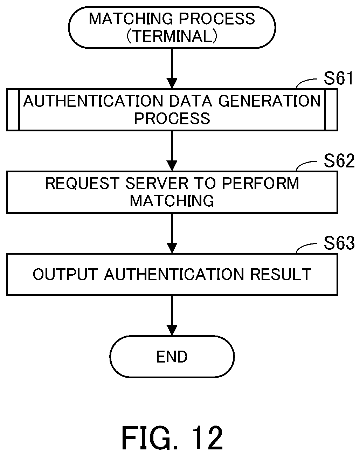

FIG. 12 is a flowchart illustrating an example of a matching process in the authentication terminal.

[Step S61] An authentication data generation process is executed based on a biometric image captured by the biometric sensor 207. The processing contents are the same as those in FIGS. 10 and 11.

[Step S62] The communication unit 260 transmits the generated authentication data to the authentication server 100 together with a corresponding user ID, and requests the authentication server 100 to execute a matching process.

[Step S63] The communication unit 260 receives an authentication result indicating success or failure of the biometric authentication from the authentication server 100. The authentication terminal 200 executes an authentication result output process. For example, the user is notified of the authentication result by an image or sound. Further, for example, a process according to the authentication result, such as unlocking of a door, may be executed.

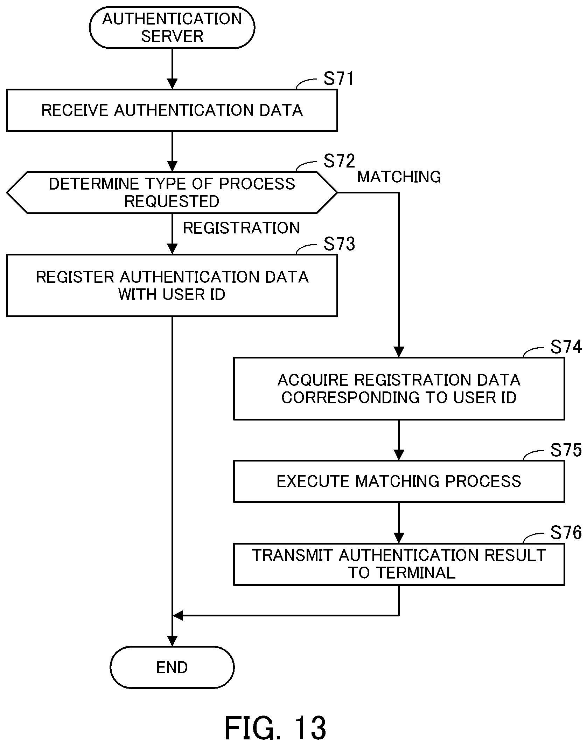

FIG. 13 is a flowchart illustrating an example of a process of the authentication server. In FIG. 13, it is assumed that one-to-one authentication is executed as an example.

[Step S71] The communication unit 120 receives the authentication data and a user ID from the authentication terminal 200.

[Step S72] The communication unit 120 determines the type of process requested. If a registration request is received together with the authentication data and the user ID in step S71, the communication unit 120 determines that the registration process is requested. In this case, the authentication data and the user ID are passed to the registration processing unit 130, and the process proceeds to step S73. On the other hand, if a matching request is received together with the authentication data and the user ID in step S71, the communication unit 120 determines that the matching process is requested. In this case, the authentication data and the user ID are passed to the matching processing unit 140, and the process proceeds to step S74.

[Step S73] The registration processing unit 130 registers the authentication data as registration data in the authentication database 111 in association with the user ID.

[Step S74] The matching processing unit 140 acquires registration data corresponding to the user ID from the authentication database 111.

[Step S75] The matching processing unit 140 matches the registration data acquired from the authentication database 111 against the authentication data (matching data) transferred from the communication unit 120. In this matching, a matching score indicating the similarity between the registration data and the matching data is calculated. When the registration data and the matching data are feature vectors, for example, a value inversely proportional to the inter-vector distance between the registration data and the matching data or the cosine similarity between the registration data and the matching data is calculated as the matching score. Then, the matching processing unit 140 compares the calculated matching score with a predetermined threshold.

[Step S76] The matching processing unit 140 transmits an authentication result based on the comparison result between the matching score and the threshold to the authentication terminal 200 via the communication unit 120. If the matching score is equal to or greater than the threshold, an authentication result indicating that the authentication has succeeded is transmitted, and if the matching score is less than the threshold, an authentication result indicating that the authentication has failed is transmitted.

### [Third Embodiment]

In the second embodiment described above, in the processing of the normalization processing unit 240 of the authentication terminal 200, the threshold Th2 indicating the upper limit of the correction amount is fixedly set. However, the threshold Th2 may be dynamically set according to the deviation amount of the posture in the normalization processing unit 220.

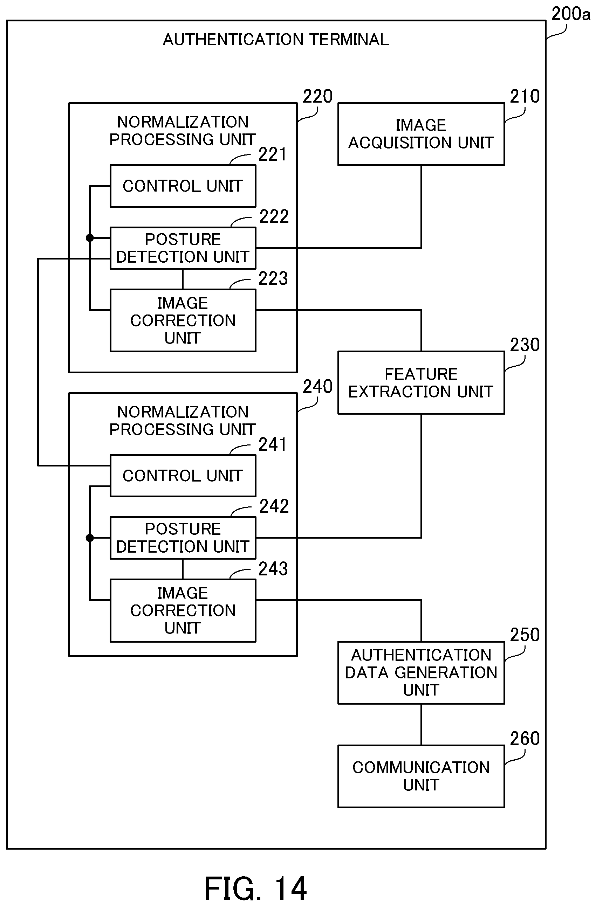

FIG. 14 is a diagram illustrating a configuration example of processing functions of an authentication terminal according to a third embodiment. In FIG. 14, components corresponding to those in FIG. 7 are denoted by the same reference numerals. In this authentication terminal 200a illustrated in FIG. 14, processes in the normalization processing units 220 and 240 are different from those in FIG. 7.

The posture detection unit 222 of the normalization processing unit 220 outputs the correction amount Θ1 indicating the deviation amount of the posture detected from the captured image to the normalization processing unit 240. The control unit 241 of the normalization processing unit 240 sets a value corresponding to the correction amount Θ1 as the threshold Th2 indicating the upper limit of the correction amount in the image correction unit 243.

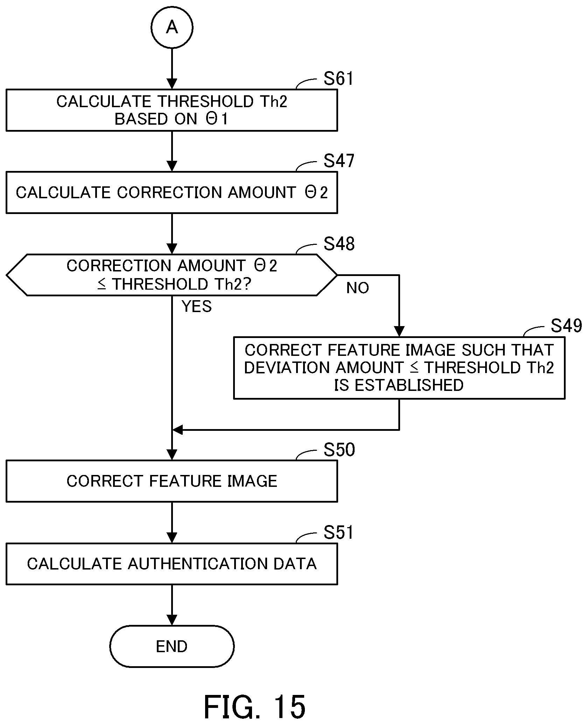

FIG. 15 is a flowchart illustrating an example of an authentication data generation process according to the third embodiment. In the authentication data generation process according to the present embodiment, a process illustrated in FIG. 15 is executed, instead of the process illustrated in FIG. 11. In FIG. 15, step S61 is executed before step S47.

[Step S61] The control unit 241 of the normalization processing unit 240 acquires the correction amount Θ1 detected by the posture detection unit 222 of the normalization processing unit 220 in step S42 in FIG. 10. The control unit 241 calculates and sets the threshold Th2 using the following equation (1) based on the acquired correction amount Θ1. $Th 2 = Θ 1 \times α \left(where 0<α<1\right)$

In practice, the angle θ1, the translation amounts (dx1, dy1), and the enlargement/reduction ratio s1 calculated in step S42 are acquired. Next, Th_θ2, Th_dx2, and Th_dy2 are calculated using a coefficient α individually set for each of θ1, dx1, and dy1. The coefficient α may be individually set within a range greater than 0 and less than 1. As to the enlargement/reduction ratio s1, the thresholds Th_s2_min and Th_s2_max may be calculated by multiplying by coefficients α1 and α2 (where 0 < α1 ≤ α2 < 0), respectively.

When the image correction amount in the normalization processing unit 220 is large, it is considered that the image correction amount in the normalization processing unit 240 is also large. This is because, in a case where the deviation amount of the posture in the captured image is large, the correction error also becomes large, and the image correction amount in the normalization processing unit 240 based on the feature image also becomes large. Therefore, it is possible to determine an appropriate value as the threshold Th2 by using the above equation (1).

### [Fourth Embodiment]

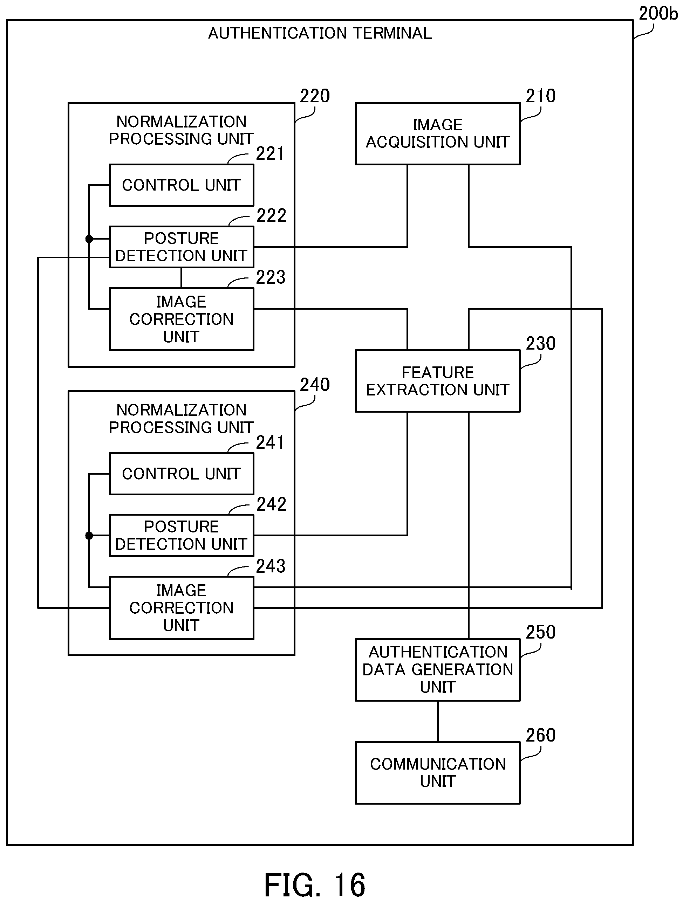

FIG. 16 is a diagram illustrating a configuration example of processing functions of an authentication terminal according to a fourth embodiment. In FIG. 16, components corresponding to those in FIG. 7 are denoted by the same reference numerals.

In the second embodiment, in the authentication terminal 200, the image correction is executed twice until the input captured image is converted into authentication data. Since a correction error may occur in both image corrections of the normalization processing units 220 and 240, when both image corrections are executed in the image processing system, the correction error may be accumulated and become large.

On the other hand, in the authentication terminal 200b according to the fourth embodiment, the image correction is executed only once in the processing system until the input captured image is converted into authentication data based on the deviation amount of the posture detected in each of the normalization processing units 220 and 240. Specifically, as in the second embodiment, the normalization processing unit 220 corrects the captured image, and the posture detection unit 242 of the normalization processing unit 240 detects the correction amount θ2 indicating the deviation amount of the posture from the feature image based on the corrected image. However, the subsequent processing is different from that of the second embodiment.

The image correction unit 243 of the normalization processing unit 240 acquires the correction amount Θ2 detected by the posture detection unit 242 and the correction amount Θ1 detected by the posture detection unit 222 of the normalization processing unit 220. Next, the image correction unit 243 corrects not the feature image but the captured image from the image acquisition unit 210 by (Θ1 + Θ2). Thereafter, a feature image is created by the feature extraction unit 230 based on the corrected captured image, and authentication data is generated by the authentication data generation unit 250 based on the feature image.

By such processing, in the processing system until the input captured image is converted into authentication data, only one image correction by the image correction unit 243 is executed. Accordingly, the correction error of the posture is suppressed, and the accuracy of the correction processing is improved. Further, the image correction is executed so as to cancel the deviation of the posture detected by both of the posture detection units 222 and 242. Therefore, highly accurate image correction is performed.

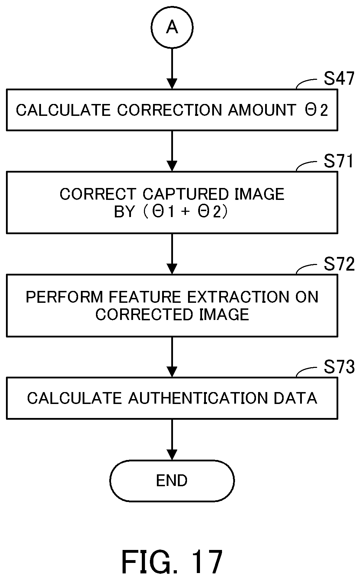

FIG. 17 is a flowchart illustrating an example of an authentication data generation process according to the fourth embodiment. In the authentication data generation process in the present embodiment, steps S71 to S73 illustrated in FIG. 17 are executed after step S47 illustrated in FIG. 11.

[Step S71] The image correction unit 243 of the normalization processing unit 240 acquires the correction amount Θ2 detected by the posture detection unit 242 and the correction amount Θ1 detected by the posture detection unit 222 of the normalization processing unit 220. The image correction unit 243 corrects the captured image from the image acquisition unit 210 by (Θ1 + Θ2).

In practice, the captured image is corrected by the rotation angle (θ1 + θ2) and the translation amounts (dx1 + dx2, dy1 + dy2). As for the scale, for example, the enlargement/reduction process is executed by the enlargement/reduction ratio (s1 × s2).

[Step S72] The feature extraction unit 230 performs a feature extraction process for extracting a vein pattern on the captured image corrected in step S71, to generate a feature image.

[Step S73] The authentication data generation unit 250 performs a predetermined calculation using the feature image generated in step S72, to calculate authentication data for registration or matching.

In the authentication data generation process according to the fourth embodiment, since the feature image generation process is executed twice, there is a high possibility that the overall processing time will be longer than that in the second and third embodiments. Therefore, for example, the authentication data generation process in the fourth embodiment may be executed at the time of registration in the authentication database 111, and the authentication data generation process in the second or third embodiment may be executed at the time of matching with the authentication database 111.

### [Fifth Embodiment]

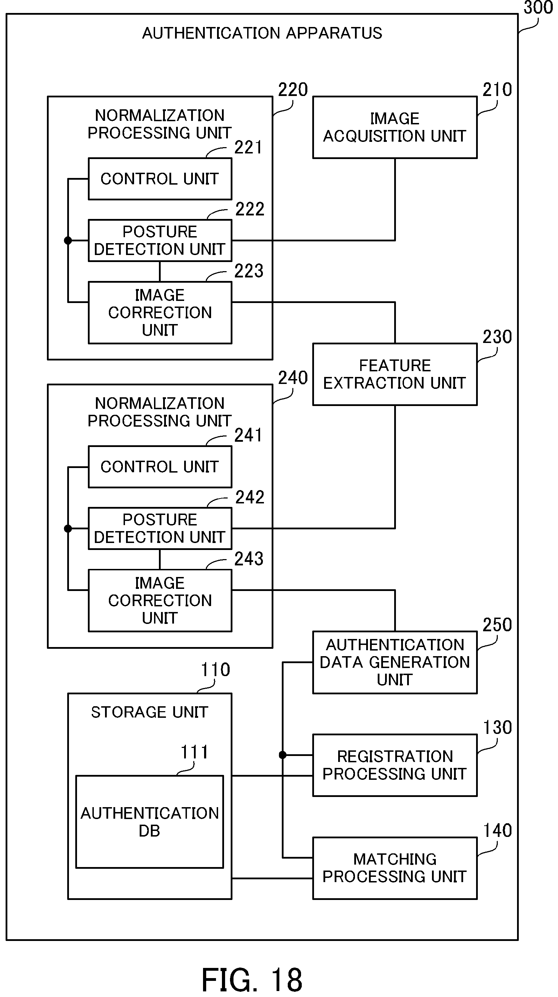

FIG. 18 is a diagram illustrating a configuration example of processing functions of an authentication apparatus according to a fifth embodiment. The processing functions of the authentication server 100 and the processing function of any one of the authentication terminals 200, 200a, and 200b may be installed in the same apparatus. The authentication apparatus 300 illustrated in FIG. 18 includes, for example, the processing functions of the authentication server 100 and the processing functions of the authentication terminal 200. In FIG. 18, components corresponding to those in FIGS. 6 and 7 are denoted by the same reference numerals.

That is, in the authentication apparatus 300, at the time of data registration, the authentication data generated by the authentication data generation unit 250 is directly transferred to the registration processing unit 130 together with a corresponding user ID. At the time of data matching, the authentication data generated by the authentication data generation unit 250 is directly transferred to the matching processing unit 140 together with a corresponding user ID.

Although the authentication apparatus 300 in FIG. 18 has the processing functions of the authentication terminal 200, the authentication apparatus 300 may instead have the processing functions of the authentication terminal 200a or the authentication terminal 200b.

The processing functions of the apparatuses described in the above embodiments (for example, the information processing apparatus 1, the authentication server 100, the authentication terminals 200, 200a, and 200b, and the authentication apparatus 300) may be implemented by a computer. In this case, a program in which the processing contents of the functions of one of the apparatuses are written is provided, and the processing functions are implemented on a computer by executing the program on the computer. The program in which the processing contents are written may be recorded in a computer-readable recording medium. Examples of the computer-readable recording medium include a magnetic storage device, an optical disc, and a semiconductor memory. Examples of the magnetic storage device include a hard disk drive (HDD) and a magnetic tape. Examples of the optical disc include a compact disc (CD), a digital versatile disc (DVD), and a Blu-ray disc (BD, registered trademark).

When the program is distributed, for example, a portable recording medium such as a DVD or a CD on which the program is recorded is sold. Alternatively, the program may be stored in a storage device of a server computer, and may be transferred from the server computer to another computer via a network.

The computer that executes the program stores, for example, the program recorded on the portable recording medium or the program transferred from the server computer in its own storage device. Then, the computer reads the program from its own storage device and executes processing according to the program. The computer may also read the program directly from the portable recording medium, and may execute processing according to the program. In addition, each time a program is transferred from the server computer connected via the network, a computer may sequentially execute processing in accordance with the received program.

The foregoing merely illustrates the principles of the invention. Further, many modifications and variations are possible for those skilled in the art, and the present invention is not limited to the exact construction and application examples illustrated and described above, and all corresponding modifications and equivalents are deemed to be within the scope of the present invention as defined by the appended claims and their equivalents.

### Reference Signs List

1 Information processing apparatus
2 Biometric sensor
3 Processing unit
11 Biometric image
12, 14 Corrected image
13 Feature image
15 Authentication data
S1 to S4 Step
θa, θb Angle

## Claims

1. An information processing program that causes a computer to execute a process comprising:
generating a first corrected image by correcting, based on a biometric image in which a predetermined body part is imaged, a posture of the body part in the biometric image;
generating a feature image by extracting a feature pattern indicating a feature of a shape of the body part from the first corrected image;
generating a second corrected image by correcting, based on the feature image, a posture of the feature pattern in the feature image; and
calculating authentication data for biometric authentication based on the second corrected image.

2. The information processing program according to claim 1, wherein a maximum correction amount of the posture used when the first corrected image is generated is set to be greater than a maximum correction amount of the posture used when the second corrected image is generated.

3. The information processing program according to claim 1, wherein the generating of the first corrected image includes:
acquiring a captured first biometric image and detecting a first deviation amount of the posture of the body part in the first biometric image from a first reference state;
acquiring a newly captured second biometric image and detecting a second deviation amount of the posture of the body part in the second biometric image from the first reference state in response to the first deviation amount exceeding a first threshold; and
generating the first corrected image by correcting the second biometric image by the second deviation amount in response to the second deviation amount being equal to or less than the first threshold.

4. The information processing program according to claim 3, wherein the generating of the first corrected image includes:
outputting a request message requesting a user whose body part has been imaged to change the posture of the body part with respect to a biometric sensor that is imaging the body part in response to the first deviation amount exceeding the first threshold.

5. The information processing program according to claim 4, wherein the generating of the second corrected image includes:
detecting a third deviation amount of the posture of the feature pattern in the feature image from a second reference state; and
generating the second corrected image by correcting the feature image by the third deviation amount in response to the third deviation amount being equal to or less than a second threshold that is less than the first threshold.

6. The information processing program according to claim 1, wherein
the generating of the first corrected image includes:
detecting a fourth deviation amount of the posture of the body part in the biometric image from a first reference state; and
generating the first corrected image by correcting the biometric image by the fourth deviation amount in response to the fourth deviation amount being equal to or less than a first threshold, and
the generating of the second corrected image includes:
setting a second threshold based on the fourth deviation amount;
detecting a fifth deviation amount of the posture of the feature pattern in the feature image from a second reference state; and
generating the second corrected image by correcting the feature image by the fifth deviation amount in response to the fifth deviation amount being equal to or less than the second threshold.

7. The information processing program according to claim 1, wherein the process further includes performing biometric authentication using the authentication data.

8. An information processing program that causes a computer to execute a process comprising:
detecting, based on a biometric image in which a predetermined body part is imaged, a first deviation amount of a posture of the body part in the biometric image from a first reference state;
generating a first corrected image by correcting the biometric image by the first deviation amount;
generating a first feature image by extracting a feature pattern indicating a feature of a shape of the body part from the first corrected image;
detecting a second deviation amount of a posture of the feature pattern in the first feature image from a second reference state;
generating a second corrected image by correcting the biometric image by a sum of the first deviation amount and the second deviation amount;
generating a second feature image by extracting the feature pattern from the second corrected image; and
calculating authentication data for biometric authentication based on the second feature image.

9. The information processing program according to claim 8, wherein the process further includes registering the authentication data in a database as registration data corresponding to a user whose body part has been imaged.

10. An information processing method executed by a computer, the information processing method comprising:
generating a first corrected image by correcting, based on a biometric image in which a predetermined body part is imaged, a posture of the body part in the biometric image;
generating a feature image by extracting a feature pattern indicating a feature of a shape of the body part from the first corrected image;
generating a second corrected image by correcting, based on the feature image, a posture of the feature pattern in the feature image; and
calculating authentication data for biometric authentication based on the second corrected image.

11. An information processing method executed by a computer, the information processing method comprising:
detecting, based on a biometric image in which a predetermined body part is imaged, a first deviation amount of a posture of the body part in the biometric image from a first reference state;
generating a first corrected image by correcting the biometric image by the first deviation amount;
generating a first feature image by extracting a feature pattern indicating a feature of a shape of the body part from the first corrected image;
detecting a second deviation amount of a posture of the feature pattern in the first feature image from a second reference state;
generating a second corrected image by correcting the biometric image by a sum of the first deviation amount and the second deviation amount;
generating a second feature image by extracting the feature pattern from the second corrected image; and
calculating authentication data for biometric authentication based on the second feature image.

12. An information processing apparatus comprising:
a processing unit for:
generating a first corrected image by correcting, based on a biometric image in which a predetermined body part is imaged, a posture of the body part in the biometric image;
generating a feature image by extracting a feature pattern indicating a feature of a shape of the body part from the first corrected image;
generating a second corrected image by correcting, based on the feature image, a posture of the feature pattern in the feature image; and
calculating authentication data for biometric authentication based on the second corrected image.

13. An information processing apparatus comprising:
a processing unit for:
detecting, based on a biometric image in which a predetermined body part is imaged, a first deviation amount of a posture of the body part in the biometric image from a first reference state;
generating a first corrected image by correcting the biometric image by the first deviation amount;
generating a first feature image by extracting a feature pattern indicating a feature of a shape of the body part from the first corrected image;
detecting a second deviation amount of a posture of the feature pattern in the first feature image from a second reference state;
generating a second corrected image by correcting the biometric image by a sum of the first deviation amount and the second deviation amount;
generating a second feature image by extracting the feature pattern from the second corrected image; and
calculating authentication data for biometric authentication based on the second feature image.
